# EUROPÄISCHE PATENTSCHRIFT

(11) **EP 0 853 944 B1**
(45) Veröffentlichungstag und Bekanntmachung des Hinweises auf die Patenterteilung: **31.03.2004**
(21) Anmeldenummer: 98890014.8
(22) Anmeldetag: 15.01.1998
(51) Int. Cl.: A61K 38/16, A61K 38/38, C07K 1/107, C07K 14/765

(54) **Präparation umfassend Thiolgruppen-haltige Proteine**
Preparation containing proteins having thiol groups
Préparations contenant des protéines à groupements thiol

(30) Priorität: 17.01.1997 AT 6897
(43) Veröffentlichungstag der Anmeldung: 22.07.1998
(73) Patentinhaber: Baxter Aktiengesellschaft, 1221 Wien (AT)
(72) Erfinder: Schlag, Günther, Prof., 1190 Wien (AT); Hallström, Seth, Dr., 1180 Wien (AT); Gasser, Harald, 1140 Wien (AT)
(74) Vertreter: Alge, Daniel, Mag. Dr. rer.nat.

(56) Entgegenhaltungen:
- WO-A-95/07691
- US-A- 4 900 719
- ZHANG Y Y ET AL: "Nitrosation of tryptophan residue(s) in serum albumin and model dipeptides. Biochemical characterization and bioactivity." JOURNAL OF BIOLOGICAL CHEMISTRY, (1996 JUN 14) 271 (24) 14271-9. , XP002114209
- SIMON D I ET AL: "Polynitrosylated proteins: characterization, bioactivity, and functional consequences." PROCEEDINGS OF THE NATIONAL ACADEMY OF SCIENCES OF THE UNITED STATES OF AMERICA, (1996 MAY 14) 93 (10) 4736-41. , XP002114210
- LAEMMLI: 'CLEAVAGE OF STRUCTURAL PROTEINS DURIND THE ASSEMBLY OF THE HEAD OF BACTERIOPHAGE T4' NATURE Bd. 227, 15 August 1970, Seiten 680 - 685

## Beschreibung

Die vorliegende Erfindung betrifft S-Nitroso-Proteinpräparationen.

NO ist ein gasförmiges Molekül, welches in vielen Säugetierzellen gebildet wird und in einer ganzen Reihe von physiologischen und pathologischen Prozessen involviert ist. So ist die Endothel-abhängige Relaxation von vaskulärer glatter Muskulatur auf NO zurückzuführen.

Der genaue Mechanismus der Wirkung von NO ist noch weitgehend unbekannt. Es wird vermutet, daß physiologische Carrier-Substanzen eine wichtige Rolle spielen.

Als mögliche derartige Carrier-Substanzen werden u.a. S-nitrosierte Proteine vermutet (Stamler et al., PNAS 89 (1992), 444-448). Es wurden daher mehrere Thiolgruppen enthaltende Proteine verschiedenster Natur und Funktion nitrosiert, wie z.B. Serumalbumin, t-PA und Kathepsin B (Stamler et al., Hallström et al. (in: Shock, Sepsis, and Organ Failure - Nitric oxide, Fourth Wiggers Bernard Conference (1994), Seiten 310-321).

Dabei wurden für bovines Serumalbumin (BSA) Nitrosierungsgrade von 85% (Stamler et al.,) bzw. 90 bis 95% für humanes Albumin (Hallström et al.) berichtet. Es zeigte sich jedoch, daß mit der in diesen Arbeiten angewendeten Methodologie zur Bestimmung der S-Nitrosobindung nicht nur die S-Nitrosierungen, sondern auch andere Nitrosierungen, wie z.B. N-Nitrosierungen an Tryptophan-Resten oder C-Nitrosierungen (an Tyrosin), detektiert werden können.

Insbesondere stellte sich die spektroskopische Messung bei 335 nm und ein molarer Extinktionskoeffizient von 3869 Mol⁻¹cm⁻¹ als nicht-spezifisch für die S-Nitrosothiolbindung heraus. U.a. konnte dieselbe Gruppe in einer Nachfolgearbeit zu Stamler et al. mit zuverlässigen Detektionsmethoden belegen, daß bei den hergestellten nitrosierten BSA-Präparaten die freien SH-Gruppen an Cystein 34 nur zu höchstens 37% nitrosiert waren (Zhang et al., Journal of Biological Chemistry 271 (24), 1996, 14271-14279).

Dieser Wert deckt sich auch mit der bekannten Tatsache, daß in Präparationen von Proteinen mit potentiell freien Thiolgruppen nur 20 bis 35% auch tatsächlich in der freien, reduzierten SH-Form vorliegen. Die übrigen 65 bis 80% sind - insbesondere bei Proteinpräparationen, welche aus Blut abgeleitet sind, oder welche im Rahmen ihres Herstellungsverfahrens mit Plasma oder Plasmaderivaten kontaktiert werden - blockiert, meist durch gemischte S-S-Bindungen mit kleinen, Thiol-tragenden Verbindungen, beispielsweise freiem L-Cystein bzw. Glutathion (Katachalski et al., J. Am. Chem. Soc. 79 (1957), 4096-4099, DeMaster et al., Biochemistry 34 (1995) 11494-11499).

Betreffend der Schwefel-hältigen Gruppierungen in den entsprechenden Proteinen ist grundsätzlich zu unterscheiden zwischen solchen, die in fest gebundener bzw. assoziierter Form, beispielsweise als intramolekular abgesättigte Disulfid-Bindungen vorliegen und die für die Konformation der Proteine entscheidend sind, und jenen, die die potentiell freie(n) Thiolgruppe(n) darstellen. Letztere sind für das jeweilige Protein eine bekannte Größe. Humanes Serumalbumin beispielsweise weist im nativen Zustand eine einzige potentiell freie Thiolgruppe pro Molekül auf, nämlich das Cystein in Position 34. Diese potentiell freien Thiolgruppen neigen jedoch zur Bildung von inter-molekularen Disulfiden, weshalb sie auch als gemischte Disulfide bezeichnet werden. In Plasma liegen bis zu 80% dieser Thiolgruppen als gemischte Disulfide vor und sind also nicht unmittelbar als freie Thiolgruppen verfügbar.

Mit Versuchen, bei welchen eine Präparation von menschlichem Serumalbumin, worin nur 20% des Cystein-34 in der reduzierten und somit freien Form vorlag, vor der Nitrosierung mit Dithiothreitol (DTT) behandelt wurde, um so die gemischten Disulfide der potentiell freien SH-Gruppe zu reduzieren, konnten abhängig von Nitrosierungsagentien und Reaktionsbedingungen unterschiedliche Nitrosierungsgrade erzielt werden (DeMaster et al.).

Ein Problem der Reduktion von potentiell freien Thiolgruppen zu freien reaktiven Thiolgruppen besteht, wenn zusätzlich auch die konformationsbestimmenden Disulfid-Bindungen aufgebrochen werden und dadurch die Nativität der Proteine verloren geht. Ein weiteres Problem entsteht bei Proteinen bzw. Proteinlösungen, die zuvor einer Behandlung zur Inaktivierung ggfs. vorhandener Pathogene, z.B. einer Hitzebehandlung, unterworfen worden sind. Dann nämlich wird i.a. eine Tendenz zur vermehrten Aggregatbildung beobachtet.

Aufgabe der vorliegenden Erfindung ist es, eine nitrosierbare bzw. eine nitrosierte Proteinpräparation sowie ein Verfahren zu deren Herstellung zur Verfügung zu stellen, deren Thiolgruppen in einem höheren Ausmaß nitrosierbar sind bzw. die einen gegenüber den beschriebenen Präparationen erhöhten S-Nitrosierungsgrad aufweist, denn je höher der Nitrosierungsgrad der Proteinpräparation ist, desto höher ist die NO-gekoppelte Wirkung, z.B. bei pharmazeutischer Verabreichung einer der artigen Proteinpräparation. Insbesondere soll eine stabile, virussichere pharmazeutische Präparation mit einem hohen S-Nitrosierungsgrad zur Verfügung gestellt werden.

Die gestellte Aufgabe wird erfindungsgemäß durch eine Präparation, umfassend Thiolgruppen-hältige Proteine, welche hitzebehandelt sind, gelöst, und die sich dadurch auszeichnen, daß sie prozessiert sind. Damit sind mindestens 40% der Thiolgruppen nitrosierbar.

Überraschenderweise wurde nämlich gefunden, daß diese virusinaktivierten Thiolgruppen-hältigen Proteinzusammensetzungen trotz Hitzebehandlung noch derart prozessiert werden können, daß die potentiell freien Thiolgruppen für weitere Reaktionen auch tatsächlich als reaktive Gruppen zur Verfügung stehen. Dadurch ist es möglich, auch bei solchen virusinaktivierten Proteinzusammensetzungen einen hohen Grad der Nitrosierung dieser Proteine zu ermöglichen, ohne die Nativität dieser Proteine nachteilig zu beeinträchtigen.

Prinzipiell kann die erfindungsgemäße pharmazeutische Präparation jegliche Proteine mit einer "freien" Thiolgruppe enthalten, bevorzugt sind aber für die Zwecke der vorliegenden Erfindung therapeutisch einsetzbare Proteine, wobei physiologische bzw. aus Blut abgeleitete humane Proteine, wie Albumin, Orosomucoid, Plasminogen-Aktivator (z.B. t-PA), Fibrinogen, Lys-Plasminogen oder Hämoglobin oder auch Mischungen derartiger erfindungsgemäß nitrosierbarer bzw. nitrosierter Proteine, als besonders bevorzugt anzusehen sind. Gemäß der vorliegenden Erfindung werden hochmolekulare Proteine im Vergleich zu niedermolekularen Proteinen wie z.B. Glutathion bevorzugt eingesetzt. Die gemäß der vorliegenden Erfindung verwendeten Proteine sind insbesondere solche, die vasodilatorische und/oder anti-aggregatorische Eigenschaften auf zelluläre Bestandteile des Blutes aufweisen. Ebenso erfindungsgemäß eingesetzt werden solche Proteine, die bei Schockzuständen eine entscheidende Rolle spielen (sogenannte Akutphasenproteine). In einer weiteren bevorzugten Ausführungsform werden Proteine eingesetzt, die sich für den Blutersatz eignen.

Mit dem erfindungsgemäßen Verfahren kann der Grad der Nitrosierung der prozessierten Thiolgruppe(n) ohne Probleme variiert werden, sogar bis zu Werten, welche einer vollständigen Nitrosierung entsprechen. Da das erfindungsgemäße Herstellungsverfahren jedoch umso zeitaufwendiger wird, je höher der Nitrosierungsgrad sein soll, wird meist zwischen Nitrosierungsgrad und Aufwand des Herstellungsverfahrens abgewogen werden müssen, sodaß bevorzugte Präparationen zu mindestens 60%, vorzugsweise zu 60 bis 95%, insbesondere zu 70 bis 80%, nitrosiert sind. Diese Werte lassen sich mit dem erfindungsgemäßen Verfahren ohne größeren Aufwand erzielen.

Die erfindungsgemäßen Präparationen können mit dem zur Verfügung gestellten Verfahren derart spezifisch S-nitrosiert werden, daß Produkte mit einem sehr niedrigen N,O,C-Nitrosierungsgrad der Proteine zur Verfügung gestellt werden können. Beispielsweise stehen N-Nitrosoverbindungen im Verdacht, krebserregend zu sein, und weisen auch eine andere Abgabekinetik der NO-Gruppe als S-Nitrosoverbindungen auf (siehe Zhang et al.), weshalb in einer bevorzugten Ausführungsform der erfindungsgemäßen Präpararation ein N,O,C-Nitrosierungsgrad der Proteine in der Präparation von unter 30, vorzugsweise unter 20%, insbesondere unter 10%, vorgesehen wird.

Obgleich es möglich ist, auch rohe Fraktionen, wie Plasma oder frühe Plasmafraktionen bzw. Kulturflüssigkeiten der erfindungsgemäßen Prozessierung bzw. Nitrosierung zu unterwerfen, werden die Proteine bevorzugterweise in gereinigter Form zur Verfügung gestellt. Der Reinheitsgrad sollte vorzugsweise geeignet sein, daß die Proteine pharmazeutisch verabreicht werden können. Daher wird die erfindungsgemäße Präparation auf Basis eines gereinigten Proteins hergestellt mit einem Reinheitsgrad von zumindest 80%, insbesondere von zumindest 90% Gew./Gew.%, bezogen auf Protein. Höhere Werte sind natürlich ebenfalls bevorzugt. Dieses gereinigte Protein kann natürlich mit weiteren Proteinen zu einer pharmazeutischen Präparation formuliert werden. Die erfindungsgemäße pharmazeutische Präparation kann also eine Mischung verschiedener nitrosierter Proteine sein, sie kann aber auch eine Mischung aus einem nitrosierten und einem nicht-nitrosierten Protein darstellen. Bevorzugterweise enthält die pharmazeutische Präparation humanes S-Nitrosoalbumin und Hämoglobin. Die erfindungsgemäße Präparation wird vorzugsweise in einer höheren Reinheit zur Verfügung gestellt, als die nicht prozessierte bzw. nicht-nitrosierte Präparation. So wird beispielsweise humanes Serumalbumin oft in einer Reinheit von mindestens 80% an Gesamtprotein als pharmazeutische Präparation verabreicht. Ein analoger oder höherer Grad der Reinheit ist daher auch für das S-nitrosierte Produkt bevorzugt. Daher wird der Nitrosierung noch ein zusätzlicher Reinigungsschritt nachgeschaltet.

Die erfindungsgemäße pharmazeutische Präparation ist vorzugsweise in einer pharmazeutisch akzeptablen Pufferlösung formuliert, gegebenenfalls mit pharmazeutisch akzeptablen Stabilisatoren. Beispielsweise wird als Stabilisator Natriumcaprylat und/oder Natriumacetyltryptophanat verwendet. In der Regel wird man dabei auf die Formulierungen, wie sie für das nicht-nitrosierte Produkt angewendet werden, zurückgreifen können. Die Präparationen können auch als Spray oder in einer für die topische Anwendung geeigneten Form zur Verfügung gestellt werden. Insbesondere wird die Präparation in einer für die intravenöse Verabreichung geeigneten Form zur Verfügung gestellt. Eine i.v.-verträgliche Präparation zeichnet sich insbesondere durch einen niedrigen Aggregatgehalt aus bzw. ist sie frei von Aggregaten. Es ist jedoch durchaus möglich, daß man für den spezifischen Einsatz der S-nitrosierten Proteine auch bei der pharmazeutischen Formulierung Vorkehrungen trifft und auch solche Substanzen zusetzt, von denen bekannt ist, daß sie die Wirkungsweise der S-nitrosierten Proteine unterstützen.

Selbstverständlich müssen auch für die Lagerung der erfindungsgemäßen Präparation oft Vorkehrungen getroffen werden, so daß diese Präparationen über einen längeren Zeitraum stabil bleiben. Die erfindungsgemäße Präparation liegt daher zu Lagerzwecken vorzugsweise in gefrorener oder lyophilisierter Form vor, in welcher sie eine ausreichend lange Haltbarkeit aufweist.

Es hat sich gezeigt, daß beispielsweise für die aus Plasma abgeleiteten Proteine, insbesondere für Albumin oder Hämoglobin, die Stabilität in Lösung bei einem pH-Wert zwischen ungefähr 6 und 7 in einem geeigneten Puffersystem (z.B. Ringerlösung) am größten ist.

Die pharmazeutische Präparation auf Basis eines Proteins mit nitrosierten Thiolgruppen gemäß der vorliegenden Erfindung ist sogar nach einer Lagerung von mehreren Tagen stabil. Die Stabilität entsprechend einer *in vivo*-Halbwertszeit konnte auch beispielsweise für humanes Albumin bewiesen werden, welches eine Halbwertszeit von wenigstens 20 Minuten, vorzugsweise 30 Minuten, insbesondere mindestens 40 Minuten bis zu 2 Stunden aufweist.

Gemäß einem weiteren Aspekt betrifft die vorliegende Erfindung ein Verfahren zur Herstellung einer erfindungsgemäßen Präparation, welches durch die folgenden Schritte gekennzeichnet ist:
- Hitzebehandeln einer Zusammensetzung umfassend Thiolgruppen-hältige Proteine,
- Bereitstellen und Prozessieren der Proteine, sodaß mindestens 40% der Thiolgrupppen nitrosierbar sind, und
- ggfs. Nitrosieren der Thiolgruppen der prozessierten Proteine in der Zusammensetzung.

Das erfindungsgemäße Verfahren unterscheidet sich in entscheidender Weise von den Verfahren gemäß dem Stand der Technik (z.B. Stamler et al.) dadurch, daß ein spezifischer Prozessierungsschritt für die Proteine vorgesehen wird, bei welchem die "freien" Thiolgruppen de-blockiert werden und somit nitrosierbar sind.

Der Prozessierungsschritt muß jedoch unter Bedingungen vorgenommen werden, bei denen die konformationsstabilisierenden Disulfidbrücken möglichst erhalten bleiben, da ansonsten auch diese u.a. zu Thiolgruppen aufgespalten werden können und bei der nachfolgenden Reaktion dann ebenfalls nitrosiert werden können. Die räumliche Struktur der Proteine sowie das Molekulargewicht werden dadurch mehr oder weniger stark beeinträchtigt. Die resultierende Heterogenität ist selbstverständlich für Proteine, welche pharmazeutisch eingesetzt werden können, möglichst zu vermeiden. Bei diagnostischen oder anderwertig einzusetzenden Proteinen kann jedoch eine (geringe) Beeinträchtigung der räumlichen Struktur der Proteine in Kauf genommen werden.

Unter Prozessierung ist erfindungsgemäß entweder die gezielte Auswahl eines Proteins, bei dem mindestens 40% der Thiolgruppen nitrosierbar sind, zu verstehen oder die Durchführung geeigneter Prozessierungsmaßnahmen, sodaß dann mindestens 40% der Thiolgruppen nitrosierbar sind.

Insbesondere bei Ausgangsmaterialien, die bereits einem Verfahren zur Inaktivierung ggfs. vorhandener Viren, insbesondere einer Hitzebehandlung, unterworfen worden sind, kann dieser Prozessierungsschritt sehr kritisch sein. Die Prozessierung darf nämlich nur in dem Ausmaß erfolgen, in dem die potentiell freien Thiolgruppen frei zugänglich gemacht werden, während die konformationsbestimmenden Disulfid-Brücken hingegen unverändert bleiben sollen.

Solche Maßnahmen können verschiedenste Verfahren umfassen, wie beispielsweise Inkubation in wäßriger Lösung bei kontrollierten Bedingungen, Inkubation mit organischen Substanzen wie z.B. β-Propiolacton, Inkubation in schwach saurem Medium, Enzymbehandlungen oder verschiedene Trennverfahren zur Abtrennung von Proteinen mit geringer Nitrosierbarkeit bzw. mit geringem Nitrosierungsgrad. Damit werden also solche Proteine, bei denen die Thiolgruppen zu mindestens 40% nitrosierbar sind, von ungeeigneten Proteinen, die dieses Kriterium nicht erfüllen, separiert. Das Ausmaß der Nitrosierbarkeit kann beispielsweise über die Bestimmung der freien Thiolgruppen ermittelt werden.

Vorzugsweise wird der erfindungsgemäße Prozessierungsschritt durch eine reduktive Behandlung vorgenommen, wobei diese mit bekannten Reduktionsmitteln, wie beispielsweise Monothiolgruppenhältigen Verbindungen, vorzugsweise β-Mercaptoethanol, vorgenommen werden kann. Das Reduktionsmittel wird im allgemeinen nach dem Gesichtspunkt ausgewählt, daß eine Reaktion mit dem entsprechenden Protein unter schonenden, nicht denaturierenden Bedingungen durchgeführt werden kann. Das Reduktionsmittel ist bevorzugterweise ein solches mit geringer Toxizität, insbesondere wird ein Mittel ausgewählt, welches nach Beenden der Reaktion mit einfachen Methoden abtrennbar ist. Deshalb wird ein Agens wie beispielsweise Dithiothreitol (DTT) in einer bevorzugten Ausführungsform nicht eingesetzt.

Die Abtrennung der Reaktionsmittel bzw. der Reaktionsprodukte erfolgt vor oder nach der Nitrosierungsreaktion und vorzugsweise in einem quantitativen Ausmaß bzw. bis zu einem Wert unter der Nachweisgrenze. Die entsprechenden für die Prozessierung verwendeten Mittel können ggfs. auch an einem festen Trägermaterial immobilisiert sein, sodaß sie nach Ablauf der gewünschten Reaktion besonders einfach entfernt werden können.

Die erfindungsgemäße Präparation zeichnet sich in einer weiteren bevorzugten Ausführungsform auch durch einen niedrigen Aggregatgehalt aus. Insbesondere liegt der Anteil an Aggregaten in der pharmazeutischen Präparation unter 20%, bevorzugterweise unter 10%, am meisten bevorzugt unter 5%.

Um beim Prozessierungsschritt insbesondere unter Verwendung eines reduzierenden Mittels spezifisch die potentiell freien Thiolgruppen de-blockieren zu können, wird dieser Schritt vorzugsweise bei einer Temperatur unter 20°C, mehr bevorzugt bei Temperaturen zwischen 2 und 10°C, und während einer Zeitdauer zwischen 1 und 100 Stunden, mehr bevorzugt während 12 bis 48 Stunden, vorgenommen. Es hat sich gezeigt, daß derart niedrige Temperaturen die Spezifität des Prozessierungsschrittes äußerst positiv beeinflussen, jedoch dauert auch die (vollständige) De-Blockierungsreaktion bei tieferen Temperaturen entsprechend länger. Es ist zwar möglich, mit einer hohen Prozessierungszeitdauer eine vollständige De-Blockierung der entsprechenden Thiolgruppen zu erzielen, es hat sich aber auch gezeigt, daß bei einer Behandlungsdauer zwischen 12 und 48 Stunden in der Regel 80 bis 90% dieser Thiolgruppen de-blockiert sind, was für viele Zwecke als ausreichend angesehen werden kann. Bei der industriellen Herstellung der erfindungsgemäßen Präparationen wird man daher immer zwischen dem zu erzielenden Nitrosierungsgrad und der Zeitdauer des Verfahrens abwägen müssen.

Von entscheidender Bedeutung für das erfindungsgemäße Verfahren ist auch, daß der Nitrosierungsschritt der prozessierten Proteine unter aeroben Bedingungen vorgenommen wird, insbesondere, wenn mit saurem Natriumnitrit gearbeitet wird.

Die erfindungsgemäße Nitrosierung wird vorzugsweise mit einem Agens, ausgewählt aus HNO₂, HNO, NOCl, NO⁺, RNO₂, N₂O₃, N₂O₄, NO₂und NO-Radikal, und in einem sauren Medium durchgeführt. Auch organische NO-Donoren können erfindungsgemäß eingesetzt werden.

Für das Ausmaß der S-Nitrosierung entscheidend ist insbesondere die Art des verwendeten Nitrosierungsagens, auch das Verhältnis des Agens bezogen auf Thiolgruppen-Gehalt des Proteins ist von Bedeutung.

Um den Grad an N-Nitrosierung möglichst gering zu halten, sollte die Nitrosierung nur mit einem relativ geringen Überschuß am entsprechenden Agens vorgenommen werden, bevorzugterweise wird das Agens für die Nitrosierung bezogen auf Thiolgruppen-Gehalt des Proteins in einem Verhältnis 0,5:1 bis 6:1, vorzugsweise in einem Verhältnis von 1:1 bis 2:1 zugesetzt. Auch sollte die Zeitdauer der Nitrosierung möglichst knapp bemessen werden, da die S-Nitrosierung schneller abläuft als die N-Nitrosierung. Bevorzugterweise wird daher die Nitrosierung während einer Zeitdauer von 2 Minuten bis mehrere Stunden, vorzugsweise 30 Minuten, bei einer Temperatur zwischen 15-30°C, vorzugsweise bei Raumtemperatur in einer wäßrigen Lösung bei pH 0,3 bis 3,5 am meisten bevorzugt bei pH 1,0 bis 3,0 vorzugsweise in saurem Bereich bis pH 5, Medium durchgeführt.

Als Ausgangsmaterial für das erfindungsgemäße Verfahren können sämtliche Arten von Proteinfraktionen verwendet werden, also insbesondere auch Blut, Plasma, Serum, eine Plasmafraktion oder eine gereinigte Proteinpräparation, aber auch Kulturüberstände oder entsprechende Extrakte. Falls jedoch Substanzen im Ausgangsmaterial enthalten sind, welche den Prozessierungs- oder den Nitrosierungsschritt negativ beeinträchtigen können, wie beispielsweise niedermolekulare Thiolgruppen-hältige Proteine, sollten diese vorzugsweise vorher abgetrennt werden. Bevorzugte Plasmafraktionen sind solche nach der Cohn-Fraktionierung und insbesondere die Cohn II- und III- Fraktion oder die Cohn IV-Fraktion.

Im Rahmen des erfindungsgemäßen Verfahrens können auch eine ganze Reihe weiterer Reinigungsschritte an beliebigen Punkten des Verfahrens vorgesehen werden.

Eine bevorzugte Ausführungsform des erfindungsgemäßen Verfahrens ist daher dadurch gekennzeichnet, daß mindestens ein weiterer Reinigungsschritt, ausgewählt aus Fällung, Gelfiltration, Diafiltration, Ultrafiltration und chromatographischer Reinigung, vorgesehen wird. Beispielsweise wird Albumin mittels einer Ionenaustauschchromatographie gereinigt.

Insbesondere kann vorgesehen werden, daß ein Reinigungsschritt nach der Prozessierung und/oder nach der Nitrosierung durchgeführt wird, so daß sich die dort eingesetzten Substanzen nicht gegenseitig beeinflussen und auch nicht in der fertigen Proteinpräparation vorhanden sind. Vorzugsweise wird dieser Reinigungsschritt in Form einer chromatographischen Reinigung, insbesondere mittels Gelpermeationschromatographie, durchgeführt.

Um die Virussicherheit zu gewährleisten, ist die erfindungsge mäße Präparation hitzebehandelt, insbesondere gemäß der EP 0 159 311-A oder der EP 0 637 451. Gemäß einer bevorzugten Ausführungsform wird eine Hitzebehandlung bei Temperaturen um 60°C für einen Zeitraum zwischen einer bis zehn Stunden durchgeführt. Zur Inaktivierung oder Abreicherung von ggfs. vorhandenden Pathogenen, insbesondere von Viren, können eine Reihe weiterer physikalischer, chemischer oder chemisch/physikalischer Verfahren durchgeführt werden. Lediglich exemplarisch sollen hier aufgezählt werden eine Hydrolasebehandlung gemäß der EP 0 247 998-A, eine Strahlenbehandlung, eine Nanofiltration oder eine Behandlung mit organischen Lösungsmitteln und/oder Tensiden, z.B. gemäß der EP 0 131 740-A. Weitere geeignete Virusinaktivierungsschritte bei der Herstellung der erfindungsgemäßen Präparate sind in der EP 0 506 651-A oder in der WO 94/13329-A beschrieben.

Die Behandlung zur Inaktivierung von Viren wird vorzugsweise bereits vor der Prozessierung durchgeführt, sie kann aber auch terminal, also anschließend an die Nitrosierung vorgenommen werden.

Nach der Nitrosierung kann das erfindungsgemäße Präparat in an sich bekannter Weise zu einem pharmazeutischen Präparat aufgearbeitet werden. In der Regel hält man sich dabei an die Formulierungsvorschriften (siehe Pharmakopöe) für die nicht-nitrosierten Proteinpräparationen.

Gemäß einem weiteren Aspekt betrifft die vorliegende Erfindung die Verwendung von Thiolgruppen-hältigen Proteinen zur Herstellung einer nitrosierten pharmazeutischen Präparation für verschiedene medizinische Verwendungen.

Bevorzugte medizinische Verwendungen dieses erfindungsgemäßen Präparates umfassen die Herstellung einer Präparation zur Verbesserung der Perfusion bzw. Mikrozirkulation, vorzugsweise in vitalen Organen wie z.B. im Gehirn (zerebrale Ischämie), im Herz (Myokardinfarkt), in der Niere, in der Leber oder in den Extremitäten bzw. im Gesamtorganismus. Damit kann die erfindungsgemäße Präparation allgemein zur Verhinderung bzw. Behandlung von Ischämie und Reperfusionsschäden eingesetzt werden. Die erfindungsgemäße Präparation ist auch zur Behandlung von Schock, insbesondere traumatischem, hypovolämischem bzw. hämorrhagischem oder neurogenem Schock geeignet.

Die Präparation gemäß der vorliegenden Erfindung kann auf verschiedenen chirurgischen Gebieten verwendet werden, beispielsweise in der Transplantationschirurgie und bei allen chirurgischen Eingriffen mit nachfolgender Reperfusion. Sie ist besonders zur Behandlung und/oder Prophylaxe einer Restenose nach Angioplastie geeignet.

Die Präparation kann auch zur Behandlung und/oder Prophylaxe thrombotischer Zustände verwendet werden, d.h. solcher, die mit einer Blutplättchenadhäsion/-aggregation verbunden sind. Bei einer bevorzugten Ausführungsform kann der S-NO-Gewebsplasminogen-Aktivator als thrombolytisches Mittel verwendet werden.

Ein weiterer Zweck der vorliegenden Präparation ist ihre Verwendung bei Bluthochdruck-Krisen.

Die Präparation ist weiters zur Relaxierung der nicht-vaskulären, glatten Muskulatur, wie z.B. der glatten Muskulatur der Luftwege, verwendbar. Daher kann die Präparation gemäß der vorliegenden Erfindung zur Behandlung und/oder Prophylaxe von Atemwegserkrankungen verwendet werden. Sie kann auch zur Diagnose und/oder Behandlung von Erektionsstörungen des Mannes nützlich sein.

Die medizinische Präparation wird vorzugsweise in einer Dosis bereitgestellt, die maximal jener des nicht-nitrosierten Proteins entspricht.

Ein Vorteil der erfindungsgemäßen nitrosierten Präparationen liegt auch darin, daß sie aufgrund ihres hohen S-Nitrosogehalts in geringeren Mengen verabreicht werden kann, als Präparationen mit einem wesentlich geringeren S-Nitrosogehalt. Von Bedeutung ist auch, daß die Abgabe der aktiven NO-Gruppe bei den erfindungsgemäß verwendeten Proteinen über einen längeren Zeitraum erfolgt und insgesamt die Kinetik dieser Reaktion unter physiologischen Bedingungen vorteilhaft ist.

Die zu verabreichende Menge bzw. Dosis hängt von den Anforderungen des Patienten ab, beispielsweise von Parametern wie Blutdruck, Hematokrit, venöser Druck oder Albumin-Level, und kann von Fall zu Fall recht unterschiedlich sein. Bei Erwachsenen beträgt die empfohlene Dosis beispielsweise 20 bis 40 g pro Tag, bei Kindern liegt sie beispielsweise zwischen 0,3 - 0,3 g/kg. Als untere Grenze wird 1 µmol/kg empfohlen. Für die Plättchenaggregations-adhäsionshemmende Wirkung kann auch eine geringere Dosis verwendet werden.

Selbstverständlich können die erfindungsgemäßen Präparate auch für jegliche Indikation der nicht-nitrosierten Proteine verwendet werden, da ihre physiologische Wirkung trotz Nitrosierung erhalten bleibt. Zusätzlich jedoch stellen Zustände, welche das Zurverfügungstellen eines erhöhten NO-Gehaltes erfordern, bevorzugte Indikationen für die erfindungsgemäßen Präparate dar.

Die vorliegende Erfindung wird an Hand der Beispiele sowie der Zeichnungsfiguren, auf die sie jedoch nicht beschränkt sein soll, näher erläutert.

Es zeigen:
Fig. 1: die S-Nitrosogradbestimmung mit HPLC/Saville-Reaktion;
Fig. 2: die S-Nitrosogradbestimmung mit ε=3869 M⁻¹cm⁻¹ bei λ=335 nm.
Fig 3: S-Nitrosogradbestimmung von S-blockierten Albumin.

### Beispiel 1:

### Herstellung einer S-nitrosierten menschlichen Serumalbuminpräparation ohne Prozessierung

Als Ausgangsmaterial wurde hitzeinaktiviertes Human Albumin 20% (Handelsname) der Firma IMMUNO AG, Wien verwendet. Die Nitrosierung des Albumins wurde mit einem 12-fachen molaren Überschuß an NaNO₂ in 0,2 mol/l HCl für eine Zeitdauer von 30 Minuten bei Raumtemperatur im ansonsten unveränderten Medium des Handelsproduktes durchgeführt. Anschließend wurde mit 1 M NaOH neutralisiert. Zur Abtrennung unerwünschter Reaktanten wurde eine präparative Gelpermeationschromatograpie unter Verwendung eines Toyopearl TSK HW 40 (F) Gels durchgeführt. Die Elution erfolgte mit bidestilliertem Wasser bei 4°C. Das nitrosierte Albumin kann lyophilisiert werden. Der S-Nitrosograd wurde wie in Beispiel 3 beschrieben bestimmt und beträgt 25%.

### Beispiel 2:

### Herstellung einer S-nitrosierten menschlichen Serumalbuminpräparation nach Prozessierung

Als Ausgangsmaterial wurde hitzeinaktiviertes Human Albumin 20% (Handelsname) der Firma IMMUNO AG, Wien verwendet.

### a) Prozessierung:

Das Ausgangsmaterial wurde mit einem 10- bis 20-fachen molaren Überschuß an β-Mercaptoethanol in einem Phosphatpuffer, bestehend aus 50 - 100 mM Natriumphosphat, 0,1 - 0,2 mM EDTA, 150 mM NaCl, versetzt und mit HCl auf pH 6,0 bis 6,2 eingestellt und für einen Zeitraum zwischen 12 - 48 Stunden bei 4°C unter Ausschluß von Sauerstoff in Stickstoff-Atmosphäre inkubiert. Dadurch konnte ein wesentlicher Teil der im Ausgangsprodukt in einem Ausmaß von bis zu 80% vorhandenen gemischten, intermolekularen Disulfide reduziert werden. Der Nachweis freier Thiolgruppen erfolgte mittels Ellmann-Reaktion (siehe Beispiel 3).

### b) Die Reinigung erfolgt wie in Beispiel 1.

### c) Nitrosierung:

Die Nitrosierung des prozessierten und gereinigten Albumins wurde mit NaNO₂ (1:1 bis 1:1,5; frei verfügbare Thiolgruppen zu NO₂⁻) in 0,2 mol/l HCl bei pH 1,5 - 2,5 für eine Zeitdauer von 15 - 30 Minuten bei Raumtemperatur durchgeführt. Anschließend wurde mit 1 M NaOH neutralisiert.

### d) Die Reinigung erfolgt wie in Beispiel 1.

Anschließend wurde das gereinigte S-Nitroso-Albumin lyophilisiert. Der S-Nitrosograd wurde wie in Beispiel 3 beschrieben bestimmt und beträgt 63%.

### Beispiel 3:

### S-Nitrosogradbestimmung mit HPLC unter Verwendung der Saville-Reaktion mit zunehmender Konzentration von Nitrosierungsagens

Die Methode basiert auf dem Prinzip der Abtrennung des Proteins vom überschüssigen Nitrosierungsagens und den Puffersubstanzen mittels einer Gelpermeationssäule (Toyopearl TSK HW-40-S). Anschließend wird die Saville-Reaktion in einer Nachsäulenderivatisierung durchgeführt (Saville B., Analyst Vol. 83, 1958, p 670-672). Dabei erfolgt eine selektive Abspaltung der NO-Gruppe vom RS-NO durch Hg²⁺. Simultan wird eine Farbreaktion mit dem entstandenen Nitrit bei 541 nm detektiert.

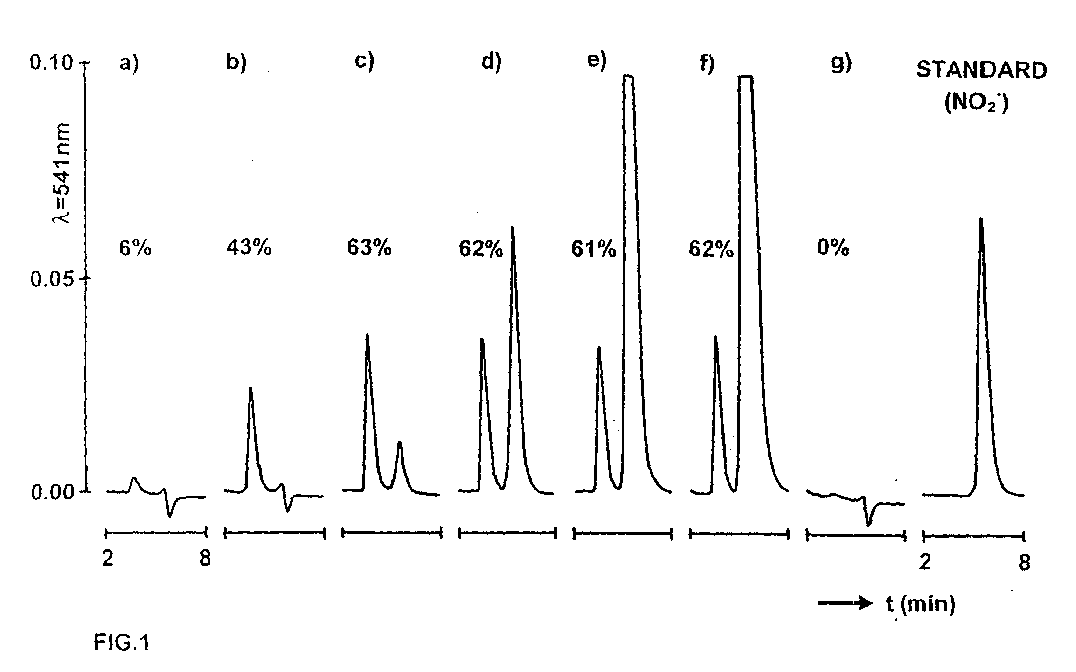

Die Chromatogramme stellen Nitrosierungen von Protein, im speziellen von HSA, mit zunehmender molarer Konzentration von Agens zu Protein (siehe Figur 1: a) 0,1; b) 0,5; c) 1; d) 2; e) 6; f) 12 und g) 0) dar. Die angeführten Prozentsätze sind, nach dem Saville-Prinzip, die tatsächlichen S-Nitrosierungsgrade am Protein. Der zweite Peak im jeweiligen Chromatogramm resultiert vom überschüssigen Nitrosierungsagens, im speziellen vom NO₂⁻.

### Beispiel 4:

### Bestimmung des S-Nitrosierungsgrades einer im Stand der Technik beschriebenen Präparation im Vergleich mit einer erfindungsgemäßen Präparation

Eine S-Nitroso-Humanalbuminpräparation wurde gemäß Stamler et al. hergestellt und mit einer erfindungsgemäß hergestellten Präparation (siehe Beispiel 2) verglichen.

Dabei wurde zunächst der S-Nitrosierungsgrad mittels des Extinktionskoeffizienten ε=3869 M⁻¹cm⁻¹ bei 335 nm für S-Nitrosothiole berechnet. Es zeigte sich, daß sich mit dieser Bestimmungsmethode für das Produkt gemäß dem Stand der Technik ein S-Nitrosograd von 90% gegenüber nur 25% für das erfindungsgemäße Produkt bestimmen ließ. Erst mit einem 12-fachen Nitrosierungsagensüberschuß konnte nach dem Verfahren von Stamler ein mittels ε bestimmter angeblicher Nitrosierungsgrad von 90% erreicht werden.

### Für beide Produkte ist das Verhältnis von freien SH-Gruppen pro Mol Protein vor der Nitrosierung mittels des Ellmann

(Ellmann G. L., Arch. Biochem. Biophys. 82, 70, 1958) bestimmt worden, wobei sich gezeigt hat, daß für das im Stand der Technik beschriebene Produkt erwartete Werte um 25% festgestellt wurden, wohingegen für das prozessierte erfindungsgemäße Produkt nach einer etwa 12-stündigen Prozessierung gemäß Beispiel 2a mit β-Mercaptoethanol bei 4°C bereits 65 - 80% der in der Präparation vorhandenen potentiell freien Thiolgruppen bereits frei waren.

Schließlich ergab die Bestimmung des S-Nitrosogrades mittels HPLC unter Verwendung der Saville-Reaktion (siehe Beispiel 3), welche sich als äußerst spezifisch für die S-Nitrosobindung herausgestellt hat, daß beim im Stand der Technik beschriebenen Produkt nur ca. 20 - 30% aller potentiellen Nitrosierungsstellen besetzt waren, wohingegen beim erfindungsgemäßen Produkt zwischen 60 und 75% der "freien" SH-Gruppen nitrosierbar sind bzw. nitrosiert waren.

Eine zusammenfassende Darstellung der Ergebnisse ist aus Tabelle 1 ersichtlich.

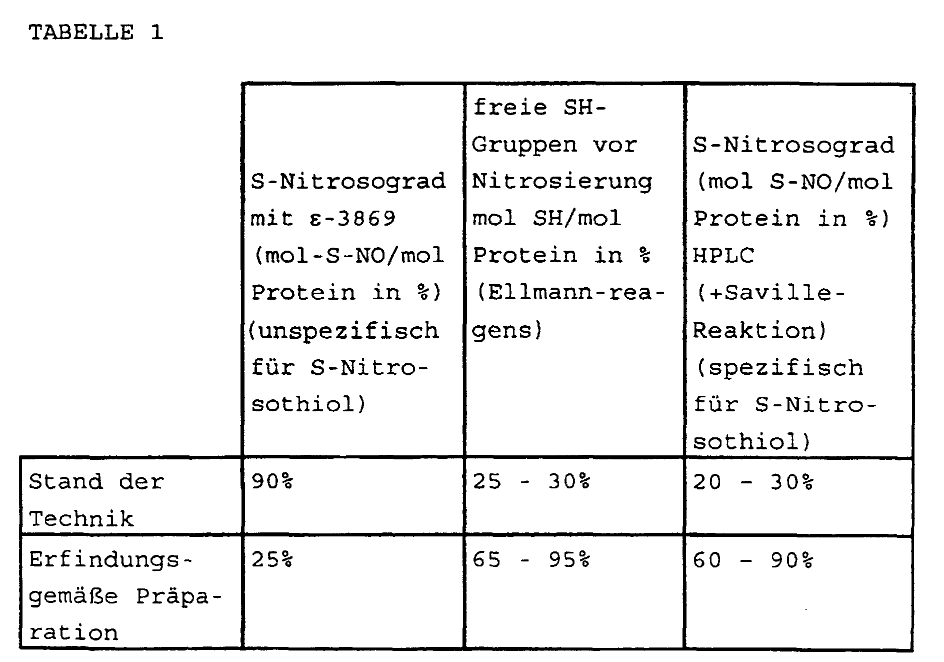

### Beispiel 5:

Vergleich der Nitrosogradbestimmung (gemäß Stamler et al.) mit der HPLC/Saville-Reaktionsbestimmung bei variierendem Überschuß an Nitrosierungs-Agens

Eine Humanserumalbuminpräparation wurde gemäß Beispiel 2 mit 0,5-, 1-, 2-, 6- und 12-fachem molarem Überschuß erfindungsgemäß nitrosiert und der S-Nitrosograd anschließend sowohl mit der Methode gemäß Stamler et al. gemessen (mit ε=3869 M⁻¹cm⁻¹ bei λ=335 nm) und parallel dazu der S-Nitrosograd mittels HPLC/Saville-Reaktion belegt.

Es zeigte sich, daß mittels der spektroskopischen Bestimmungsmethode gemäß Stamler et al., für den 1-, 2-, 6- und 12-fachen Überschuß jeweils unterschiedliche S-Nitrosograde der Präparationen bestimmt werden konnten (20%, 39%, 69% und 111%), wohingegen der Wert des mittels HPLC bestimmten S-Nitrosierungsgrades bei sämtlichen Reaktionen zwischen 60 und 65% lag.

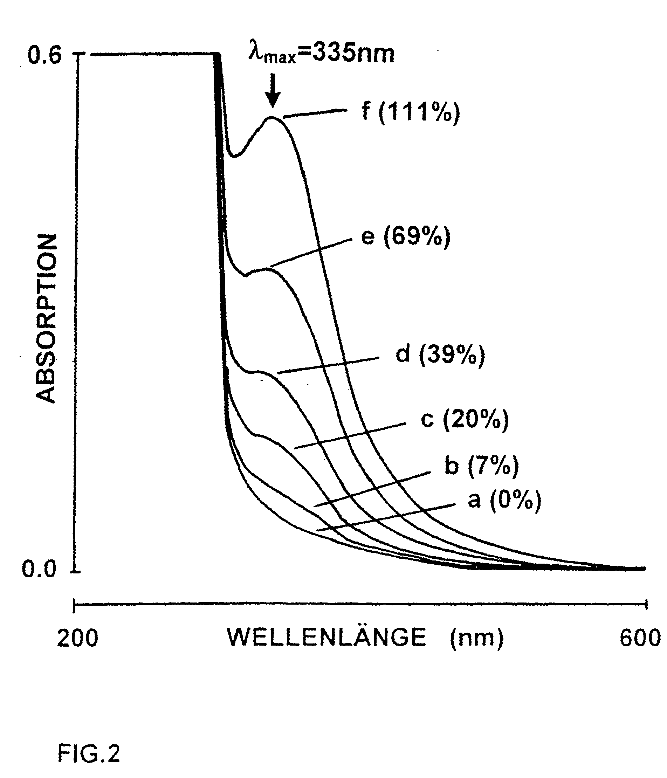

Die Ergebnisse sind zusammenfassend in Tabelle 2 sowie in den Figuren 1 und 2 dargestellt.

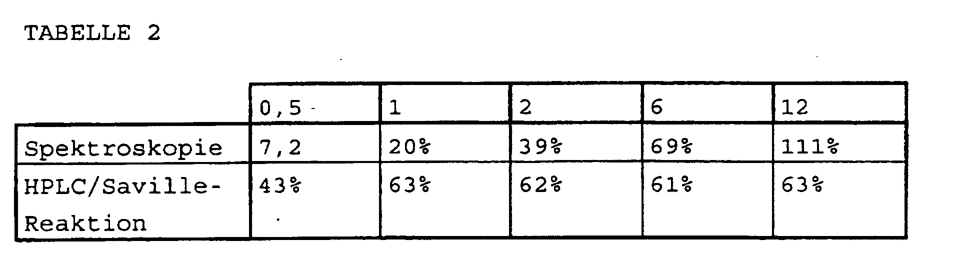

Diese Ergebnisse zeigen die Unbrauchbarkeit der Methodik zur S-Nitrosogradbestimmung, wie sie im Stand der Technik gemäß Stamler et al. und Hallström et al. verwendet worden ist. Folglich haben die dort angegebenen S-Nitrosierungsgrade nicht 85 oder 90 - 95%, sondern liegen lediglich in der Größenordnung zwischen 25 - 37%.

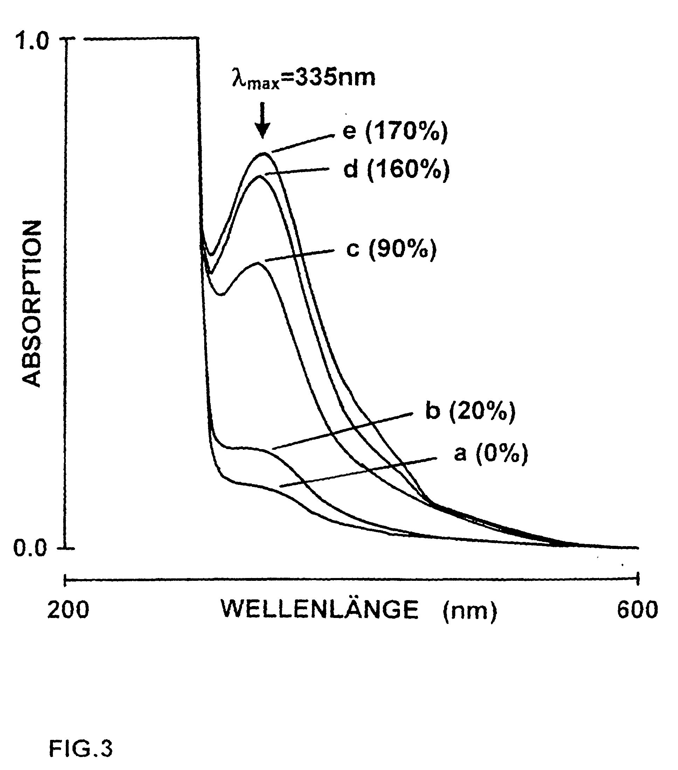

Zum Vergleich ist in in Figur 3 die spektrophotometrische Bestimmung des Nitrosierungsgrades eines modifizierten humanen Serumalbumins, bei dem die freie Thiolgruppe (Cys 34) durch Carboxymethylierung chemisch blockiert wurde, dargestellt. Die Nitrosierung erfolgte mit steigender molarer Konzentration an Nitrosierungsagens zu Protein (a) 0; b) 1; c) 6; d) 12 und e)20).

Mittels der HPLC-Nachsäulenderivatisierungsmethode konnte keine S-Nitrosierung nachgewiesen werden (S-Nitrosierungsgrad ( 1%).

## Patentansprüche

1. Verfahren zur Herstellung einer pharmazeutischen Präparation eines nitrosierten Thiolgruppen-hältigen Proteins, **gekennzeichnet durch** die folgenden Schritte
- Bereitstellen einer Thiolgruppen-hältigen Protein-Präparation
- Prozessieren der Proteine in der Präparation **durch** Behandeln der Proteine mit einem Reduktionsmittel und
- Nitrosieren der Thiolgruppen der prozessierten Proteine mit einem Agens für die Nitrosierung, wobei das Agens für die Nitrosierung, bezogen auf den Thiolgruppen-Gehalt des Proteins in einem Verhältnis von 0,5:1 bis 6:1 zugesetzt wird.

2. Verfahren nach Anspruch 1, **dadurch gekennzeichnet, dass** das Reduktionsmittel eine Monothiolgruppen-hältige Verbindung ist.

3. Verfahren nach Anspruch 1 oder 2, **dadurch gekennzeichnet, dass** das Reduktionsmittel β-Mercaptoethanol ist.

4. Verfahren nach einem der Ansprüche 1 bis 3, **dadurch gekennzeichnet, dass** die Prozessierung bei einer Temperatur unter 20°C, vorzugsweise bei Temperaturen zwischen 2 und 10°C, und während einer Zeitdauer zwischen einer und 100 Stunden, vorzugsweise zwischen 12 und 48 Stunden, vorgenommen wird.

5. Verfahren nach einem der Ansprüche 1 bis 4, **dadurch gekennzeichnet, dass** Plasma, Serum, eine Plasmafraktion oder eine gereinigte Protein-Präparation bereitgestellt werden.

6. Verfahren nach einem der Ansprüche 1 bis 5, **dadurch gekennzeichnet, dass** mindestens ein weiterer Reinigungsschritt ausgewählt aus Fällung, Gelfiltration, Ultrafiltration und chromatographische Reinigung vorgesehen wird.

7. Verfahren nach einem der Ansprüche 1 bis 6, **dadurch gekennzeichnet, dass** mindestens ein Reinigungsschritt, vorzugsweise eine chromatographische Reinigung, nach der Prozessierung und/oder nach der Nitrosierung durchgeführt wird.

8. Verfahren nach einem der Ansprüche 1 bis 7, **dadurch gekennzeichnet, dass** die Nitrosierung unter aeroben Bedingungen mit einem Agens, ausgewählt aus HNO₂, HNO, NOCl, NO⁺, RNO₂, N₂O₃, N₂O₄, NO₂- und NO-Radikal, in einem sauren Medium durchgeführt wird.

9. Verfahren nach einem der Ansprüche 1 bis 8, **dadurch gekennzeichnet, dass** das Agens für die Nitrosierung bezogen auf Thiolgruppen-Gehalt des Proteins in einem Verhältnis von 1:1 bis 2:1 zugesetzt wird.

10. Verfahren nach einem der Ansprüche 1 bis 9, **dadurch gekennzeichnet, dass** ein Schritt zur Inaktivierung oder Abreicherung von gegebenenfalls vorhandenen Pathogenen, insbesondere Viren, vorgesehen wird.

11. Verfahren nach Anspruch 10, **dadurch gekennzeichnet, dass** die Präparation hitzebehandelt wird, vorzugsweise vor der Prozessierung.

12. Stabile, virussichere pharmazeutische Präparation, umfassend nitrosierte Thiolgruppen-hältige Proteine, **dadurch gekennzeichnet, dass** zumindestens 40% der Thiolgruppen nitrosiert sind.

13. Pharmazeutische Präparation nach Anspruch 12, **dadurch gekennzeichnet, dass** die Proteine humane Proteine sind, insbesondere Proteine ausgewählt aus Albumin, Orosomucoid, Plasminogen Aktivator, insbesondere t-PA, Fibrinogen, Lys-Plasminogen, Hämoglobin oder eine Mischung davon.

14. Pharmazeutische Präparation nach Anspruch 12 oder 13, **dadurch gekennzeichnet, dass** die Thiolgruppen dieser Proteine zu mindestens 60%, vorzugsweise zu 65 bis 95%, insbesondere zu 70 bis 90%, nitrosiert sind.

15. Pharmazeutische Präparation nach einem der Ansprüche 12 bis 14, **dadurch gekennzeichnet, dass** humanes S-Nitrosoalbumin und Hämoglobin enthalten sind.

16. Pharmazeutische Präparation nach einem der Ansprüche 12 bis 15, **dadurch gekennzeichnet, dass** der N, O, C-Nitrosierungsgrad der Proteine in der Präparation unter 30%, vorzugsweise unter 20%, insbesondere unter 10%, liegt.

17. Pharmazeutische Präparation nach einem der Ansprüche 12 bis 16, **dadurch gekennzeichnet, dass** die Thiolgruppen-hältigen Proteine in gereinigter Form, vorzugsweise in einem Anteil von zumindest 80%, insbesondere von zumindest 90%, am Gesamtprotein vorliegen.

18. Pharmazeutische Präparation nach einem der Ansprüche 12 bis 17, **dadurch gekennzeichnet, dass** sie eine Stabilität entsprechend einer in vivo-Halbwertszeit von wenigstens 20 min, vorzugsweise mindestens 40 min bis 2 h, aufweist.

19. Pharmazeutische Präparation nach einem der Ansprüche 12 bis 18, **dadurch gekennzeichnet, dass** sie in gefrorener oder lyophilisierter Form vorliegt.

20. Verwendung einer Prozessierung von Thiolgruppen-hältigen Proteinen durch Behandeln der Proteine mit einem Reduktionsmittel zur Herstellung einer Präparation von Proteinen, deren Thiolgruppen zu mindestens 40% nitrosierbar sind im Rahmen der Herstellung von nitrosierten, Thiolgruppen-hältigen Proteinen.

21. Verwendung nach Anspruch 20, **dadurch gekennzeichnet, dass** neben der Prozessierung ein oder mehrere Maßnahmen der Ansprüche 2 bis 6 und 10 und 11 vorgesehen werden.

22. Verwendung nach Anspruch 20 oder 22, **dadurch gekennzeichnet, dass** mindestens ein Reinigungsschritt, vorzugsweise eine chromatographische Reinigung, nach der Prozessierung durchgeführt wird.

23. Verwendung einer stabilen, virussicheren pharmazeutischen Präparation, umfassend nitrosierbare Thiolgruppen-hältige Proteine, wie in einem der Ansprüche 20 bis 22 definiert, zur Herstellung von nitrosierten, Thiolgruppen-hältigen Proteinen.

24. Verwendung nach Anspruch 23, **dadurch gekennzeichnet, dass** die Proteine humane Proteine sind, insbesondere Proteine ausgewählt aus Albumin, Orosomucoid, Plasminogen Aktivator, insbesondere t-PA, Fibrinogen, Lys-Plasminogen, Hämoglobin oder eine Mischung davon.

25. Verwendung nach Anspruch 23 oder 24, **dadurch gekennzeichnet, dass** die stabile pharmazeutische Präparation in gefrorener oder lyophilisierter Form vorliegt.

26. Verwendung von Thiolgruppen-hältigen Proteinen bzw. einer Präparation nach einem der Ansprüche 12 bis 19 zur Herstellung einer Präparation zur Verbesserung der Perfusion bzw. Mikrozirkulation.

27. Verwendung von Thiolgruppen-hältigen Proteinen bzw. einer Präparation nach einem der Ansprüche 12-19 zur Herstellung einer Präparation zur Verbesserung der Perfusion bzw. Mikrozirkulation im Gehirn, vorzugsweise bei zerebraler Ischämie, im Herz, vorzugsweise bei Myokardinfarkt, in der Niere, in der Leber und in den Extremitäten.

28. Verwendung von Thiolgruppen-hältigen Proteinen oder einer Präparation nach einem der Ansprüche 12-19 zur Herstellung einer Präparation zur Behandlung von Schock, insbesondere traumatischem Schock.

29. Verwendung nach einem der Ansprüche 26 bis 28, **dadurch gekennzeichnet, dass** die Präparation in einer Dosis bereitgestellt wird, die maximal jener des nicht nitrosierten Proteins entspricht.

## Claims

1. A method for producing a pharmaceutical preparation of a nitrosated, thiol-group-containing protein, **characterised by** the following steps
- providing a thiol-group-containing protein preparation,
- processing the proteins in the preparation by treating the proteins with a reducing agent, and
- nitrosating the thiol groups of the processed proteins with an agent for the nitrasation, said agent for said nitrosation being added in a ratio of from 0.5:1 to 6:1, based on the thiol-group content of the protein.

2. A method according to claim 1, **characterised in that** the reducing agent is a monothiol-group-containing compound.

3. A method according to claim 1 or 2, **characterised in that** the reducing agent is β-mercapto-ethanol.

4. A method according to any one of claims 1 to 3, **characterised in that** said processing is effected at a temperature of below 20°C, preferably at temperatures of between 2 and 10°C, and for a period of between one and 100 hours, preferably for a period of between 12 and 48 hours.

5. A method according to any one of claims 1 to 4, **characterised in that** plasma, serum, a plasma fraction or a purified protein preparation is provided.

6. A method according to any one of claims 1 to 5, **characterised in that** at least one further purification step selected from precipitation, gel filtration, ultrafiltration and chromatographic purification is provided.

7. A method according to any one of claims 1 to 6, **characterised in that** at least one purification step, preferably a chromatographic purification, is carried out after said processing and/or after said nitrosation.

8. A method according to any one of claims 1 to 7, **characterised in that** the nitrosation is carried out under aerobic conditions with an agent selected from HNO₂, HNO, NOCl, NO⁺, RNO₂, N₂O₃, N₂O₄, NO₂- and NO-radical, in an acidic medium.

9. A method according to any one of claims 1 to 8, **characterised in that** the agent for said nitrosation is added in a ratio of from 1:1 to 2:1, based on the thiol group content of the protein.

10. A method according to any one of claims 1 to 9, **characterised in that** a step for inactivating or depleting possibly present pathogens, in particular viruses, is provided.

11. A method according to claim 10, **characterised in that** the preparation is heat treated, preferably prior to said processing.

12. A stable, virus-safe pharmaceutical preparation comprising nitrosated, thiol-group-containing proteins, **characterised in that** at least 40% of said thiol groups are nitrosated.

13. A pharmaceutical preparation according to claim 12, **characterised in that** the proteins are human proteins, in particular proteins selected from albumin, orosomucoid, plasminogen activator, in particular t-PA, fibrinogen, Lys-plasminogen, haemoglobin or a mixture thereof.

14. A pharmaceutical preparation according to claim 12 or 13, **characterised in that** at least 60%, preferably 65 to 95%, in particular 70 to 90% of the thiol groups of these proteins are nitrosated.

15. A pharmaceutical preparation according to any one of claims 12 to 14, **characterised in that** human S-nitroso-albumin and haemoglobin are contained therein.

16. A pharmaceutical preparation according to any one of claims 12 to 15, **characterised in that** the N, O, C-nitrosation level of the proteins in the preparation is below 30%, preferably below 20%, in particular below 10%.

17. A pharmaceutical preparation according to any one of claims 12 to 16, **characterised in that** the thiol-group-containing proteins are provided in purified form, preferably in an amount of at least 80%, in particular of at least 90% of the total protein content.

18. A pharmaceutical preparation according to any one of claims 12 to 17, **characterised in that** it has a stability corresponding to an in vivo half-life of at least 20 min, preferably at least 40 min to 2 h.

19. A pharmaceutical preparation according to any one of claims 12 to 18, **characterised in that** it is provided in frozen or lyophilized form.

20. The use of a processing of thiol-group-containing proteins by treating the proteins with a reducing agent for producing a preparation of proteins whose thiol groups can be nitrosated by at least 40% within the scope of the production of nitrosated, thiol-group-containing proteins.

21. The use according to claim 20, **characterised in that** one or more measures of claims 2 to 6 and 10 and 11 are provided besides said processing.

22. The use according to claim 20 or 22, **characterised in that** at least one purification step, preferably a chromatographic purification, is carried out after said processing.

23. The use of a stable, virus-safe pharmaceutical preparation, comprising thiol-group-containing proteins capable of being nitrosated, as defined in any one of claims 20 to 22, for producing nitrosated, thiol-group-containing proteins.

24. The use according to claim 23, **characterised in that** the proteins are human proteins, in particular proteins selected from albumin, orosomucoid, plasminogen activator, in particular t-PA, fibrinogen, Lys-plasminogen, haemoglobin or a mixture thereof.

25. The use according to claim 23 or 24, **characterised in that** the stable pharmaceutical preparation is provided in frozen or lyophilized form.

26. The use of thiol-group-containing proteins or of a preparation according to any one of claims 12 to 19, respectively, for producing a preparation for improving the perfusion and microcirculation, respectively.

27. The use of thiol-group-containing proteins or of a preparation according to any one of claims 12 to 19, respectively, for producing a preparation for improving the perfusion and microcirculation, respectively, in the brain, preferably in case of cerebral ischemia, in the heart, preferably in case of myocardial infarct, in the kidney, in the liver and in the extremities.

28. The use of thiol-group-containing proteins or of a preparation according to any one of claims 12 to 19 for producing a preparation for the treatment of shock, in particular traumatic shock.

29. The use according to any one of claims 26 to 28, **characterised in that** the preparation is provided in a dose corresponding to maximal that of the non-nitrosated protein.

## Revendications

1. Procédé de fabrication d'une préparation pharmaceutique d'une protéine à groupements thiol nitrosée, **caractérisé par** les étapes suivantes
- Mise à disposition d'une préparation de protéine contenant des groupements thiols
- Transformation des protéines dans la préparation par traitement des protéines avec un réducteur et
- Nitrosation des groupements thiol des protéines manipulées avec un agent pour la nitrosation, l'agent pour la nitrosation étant ajouté dans un ratio de 0,5 : 1 à 6 : 1 par rapport à la teneur en groupements thiol de la protéine.

2. Procédé selon la revendication 1, **caractérisé en ce que** le réducteur est un composé contenant un groupement monothiol.

3. Procédé selon la revendication 1 ou 2, **caractérisé en ce que** le réducteur est le β-mercaptoéthanol.

4. Procédé selon l'une quelconque des revendications 1 à 3, **caractérisé en ce que** la transformation est effectuée à une température inférieure à 20°C, de préférence à des températures comprises entre 2 et 10°C, et pendant une durée comprise entre une et 100 heures, de préférence entre 12 et 48 heures.

5. Procédé selon l'une quelconque des revendications 1 à 4, **caractérisé en ce que** du plasma, du sérum, une fraction de plasma ou une préparation de protéine purifiée est mise à disposition.

6. Procédé selon l'une quelconque des revendications 1 à 5, **caractérisé en ce qu'**au moins une étape de purification supplémentaire choisie parmi la précipitation, la filtration de gel, l'ultrafiltration et la purification par chromatographie est prévue.

7. Procédé selon l'une quelconque des revendications 1 à 6, **caractérisé en ce qu'**au moins une étape de purification, de préférence une purification par chromatographie est réalisée après la transformation et/ou après la nitrosation.

8. Procédé selon l'une quelconque des revendications 1 à 7, **caractérisé en ce que** la nitrosation est réalisée en milieu acide dans des conditions aérobies avec un agent choisi parmi HNO₂, HNO, NOCl, NO⁺, RNO₂, N₂O₃, N₂O₄, NO₂⁻, et le radical NO.

9. Procédé selon l'une quelconque des revendications 1 à 8, **caractérisé en ce que** l'agent pour la nitrosation est ajouté dans un ratio de 1 : 1 à 2 : 1 par rapport à la teneur en groupements thiol de la protéine

10. Procédé selon l'une quelconque des revendications 1 à 9, **caractérisé en ce qu'**une étape est prévue pour inactiver ou appauvrir des pathogènes présents le cas échéant, en particulier des virus.

11. Procédé selon la revendication 10, **caractérisé en ce que** la préparation est traitée par chauffage, de préférence avant la transformation.

12. Préparation pharmaceutique stable protégée des virus comportant des protéines à groupements thiol nitrosées, caractérisée en ce que les groupements thiol sont nitrosés au moins à 40 %.

13. Préparation pharmaceutique selon la revendication 12, **caractérisée en ce que** les protéines sont des protéines humaines, en particulier des protéines choisies parmi l'albumine, l'orosomucoïde, un activateur plasminogène, en particulier le t-PA, un fibrinogène, le lys-plasminogène, l'hémoglobine ou un mélange de ceux-ci.

14. Préparation pharmaceutique selon la revendication 12 ou 13, **caractérisée en ce que** les groupements thiol de ces protéines sont nitrosés au moins à 60 %, de préférence de 65 à 95 %, en particulier de 70 à 90 %.

15. Préparation pharmaceutique selon l'une quelconque des revendications 12 à 14, **caractérisée en ce que** de la S-Nitroalbumine humaine et de l'hémoglobine sont contenues.

16. Préparation pharmaceutique selon l'une quelconque des revendications 12 à 15, **caractérisée en ce que** le degré de nitrosation N, O, C, des protéines dans la préparation se situe en dessous de 30 %, de préférence en dessous de 20 %, en particulier en dessous de 10 %.

17. Préparation pharmaceutique selon l'une quelconque des revendications 12 à 16, **caractérisée en ce que** les protéines à groupements thiol sont disponibles dans la protéine globale sous forme purifiée, de préférence dans une proportion d'au moins 80 %, en particulier d'au moins 90 %.

18. Préparation pharmaceutique selon l'une quelconque des revendications 12 à 17, **caractérisée en ce qu'**elle présente une stabilité correspondant à une période de demi-vie in-vivo d'au moins 20 mn, de préférence au moins 40 mn à 2 h.

19. Préparation pharmaceutique selon l'une quelconque des revendications 12 à 18, **caractérisée en ce qu'**elle est disponible sous forme congelée ou lyophilisée.

20. Utilisation d'une transformation de protéines à groupements thiol par traitement des protéines avec un réducteur pour la fabrication d'une préparation de protéines dont les groupements thiol peuvent être nitrosés à au moins 40 % dans le cadre de la préparation de protéines à groupements thiol nitrosées.

21. Utilisation selon la revendication 20, **caractérisée en ce qu'**en plus de la transformation, une ou plusieurs mesures des revendications 2 à 6 et 10 et 11 sont prévues.

22. Utilisation selon la revendication 20 ou 21, caractérisée en ce qu'au moins une étape de purification, de préférence une purification par chromatographie, est réalisée après la transformation.

23. Utilisation d'une préparation pharmaceutique stable protégée des virus comportant des protéines à groupements thiol nitrosées comme définies dans l'une quelconque des revendications 20 à 22 pour la préparation de protéines à groupements thiols nitrosées.

24. Utilisation selon la revendication 23, **caractérisée en ce que** les protéines sont des protéines humaines, en particulier des protéines choisies parmi l'albumine, l'orosomucoïde, un activateur plasminogène, en particulier le t-PA, un fibrinogène, le lys-plasminogène, l'hémoglobine ou un mélange de ceux-ci.

25. Utilisation selon la revendication 23 ou 24, caractérisée en ce que la préparation pharmaceutique stable est disponible sous forme congelée ou lyophilisée.

26. Utilisation de protéines à groupements thiol et/ou d'une préparation selon l'une quelconque des revendications 12 à 19 pour la fabrication d'une préparation pour améliorer la perfusion et/ou la microcirculation.

27. Utilisation de protéines à groupements thiol et/ou d'une préparation selon l'une quelconque des revendications 12 à 19 pour la fabrication d'une préparation pour améliorer la perfusion et/ou la microcirculation dans le cerveau, de préférence dans le cas d'une ischémie cérébrale, dans le coeur, de préférence dans le cas d'un infarctus du myocarde, dans les reins, dans le foie et dans les extrémités.

28. Utilisation de protéines à groupements thiols ou d'une préparation selon l'une quelconque des revendications 12 à 19 pour la fabrication d'une préparation pour le traitement d'un choc, en particulier d'un choc traumatique.

29. Utilisation selon l'une quelconque des revendications 26 à 28, **caractérisée en ce que** la préparation est mise à disposition dans une dose qui correspond au maximum à celle de la protéine non-nitrosée.
